# EUROPÄISCHE PATENTSCHRIFT

(11) **EP 0 556 683 B1**
(45) Veröffentlichungstag und Bekanntmachung des Hinweises auf die Patenterteilung: **24.04.1996**
(21) Anmeldenummer: 93101948.3
(22) Anmeldetag: 08.02.1993
(51) Int. Cl.: C07D 213/61

(54) **Verfahren zur Herstellung von 2-Chlor-5-methyl-pyridin**
Process for the preparation of 2-chloro-5-methylpyridine
Procédé pour la préparation de 2-chloro-5-méthyl-pyridine

(30) Priorität: 19.02.1992 DE 4204920; 15.04.1992 DE 4212595
(43) Veröffentlichungstag der Anmeldung: 25.08.1993
(73) Patentinhaber: BAYER AG, D-51368 Leverkusen (DE)
(72) Erfinder: Rivadeneira, Eric, Dr., W-5090 Leverkusen 3 (DE); Jelich, Klaus, Dr., W-5600 Wuppertal 1 (DE)

(56) Entgegenhaltungen:
- EP-A- 0 438 691
- EP-A- 0 439 745
- EP-A- 0 463 464
- CHEMICAL ABSTRACTS, vol. 89, no. 25, 18. Dezember 1978, Columbus, Ohio, US; abstract no. 215188r, A.R. KATRITZKY ET AL. 'Preparation of some novel pyridone derivatives.' Seite 574 ;
- JOURNAL OF THE CHEMICAL SOCIETY, PERKIN TRANSACTIONS 1 Nr. 11, 1983, LETCHWORTH GB Seiten 2623 - 2627 A.R. KATRITZKY ET AL. 'Nucleophilic displacement of N-aryl and heteroaryl groups. Part 5. Conversion of 2-aminopyridines into 2-pyridones.'
- JOURNAL OF THE CHEMICAL SOCIETY, PERKIN TRANSACTIONS 1 Nr. 12, 1977, LETCHWORTH GB Seiten 1436 - 1445 A.S. AFRIDI ET AL. 'Synthesis, spectroscopic properties, and chemistry of 4,6-diphenyl-2-pyridones and 4,6-diphenylpyridine-2-thiones and their relationship to isomeric species.'

## Beschreibung

Die Erfindung betrifft ein neues Verfahren und neue Zwischenprodukte zur Herstellung von 2-Chlor-5-methyl-pyridin.

Es ist bekannt, daß man 2-Chlor-5-methyl-pyridin erhält, wenn man 3-Methyl-pyridin-1-oxid mit Phosgen in Gegenwart von Triethylamin umsetzt (vgl. EP-A 439 745, Beispiel 3). Die bei diesem Verfahren erzielte Ausbeute an 2-Chlor-5-methyl-pyridin ist jedoch meist unbefriedigend und das Produkt wird immer in Mischung mit einer erheblichen Menge an 2-Chlor-3-methyl-pyridin erhalten.

Es wurde nun ein neues Verfahren zur Herstellung von 2-Chlor-5-methyl-pyridin der Formel (I) 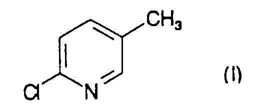 gefunden, welches dadurch gekennzeichnet ist, daß man in einer ersten Stufe 3-Methyl-pyridin-1-oxid der Formel (II) 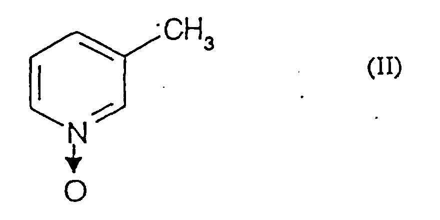 mit Trimethylamin und einer elektrophilen Verbindung aus der Reihe Phosgen, Thionylchlorid, Sulfurylchlorid, Benzolsulfonsäurechlorid und p-Toluolsulfonsäurechlorid gegebenenfalls in Gegenwart eines Verdünnungsmittels,
zur Verbindung der allgemeinen Formel (III) 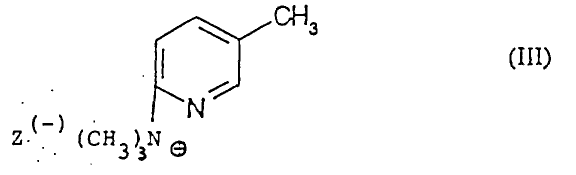 in welcher
- Z^{^{⊖}}: für ein aus der elektrophilen Verbindung gebildetes Anion steht, umsetzt,
und diese Verbindung (III) gegebenenfalls
als Rohzwischenprodukt isoliert oder gegebenenfalls weiter reinigt,
und in einer zweiten Stufe mit Chlorwasserstoff als Chlorierungsmittel gegebenenfalls in Gegenwart eines Alkylchlorids und gegebenenfalls in Gegenwart eines Verdünnungsmittels bei Temperaturen zwischen 10°C und 150°C umsetzt.

Gegenstand der Erfindung ist ferner die Verbindung folgender Formel

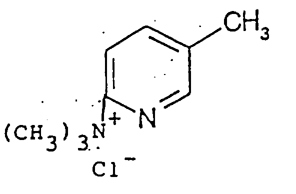

Überraschenderweise kann nach dem erfindungsgemäßen Verfahren 2-Chlor-5-methyl-pyridin der Formel (I) in hoher Ausbeute (bezogen auf umgesetztes Zwischenprodukt) und in erheblich verbesserter Isomerenreinheit im Vergleich mit der bekannten Verfahrensweise erhalten werden. Nicht umgesetztes Zwischenprodukt der Formel (III) kann erneut in den Prozeß eingesetzt werden. Das erfindungsgemäße Verfahren stellt somit eine wertvolle Bereicherung des Standes der Technik dar.

Verwendet man als organische Stickstoffbase A Trimethylamin, in der ersten Stufe Phosgen als elektrophile Verbindung und in der zweiten Stufe Phosgen als Chlorierungsmittel, so kann der Reaktionsablauf beim erfindungsgemäßen Verfahren durch das folgende Formelschema skizziert werden:

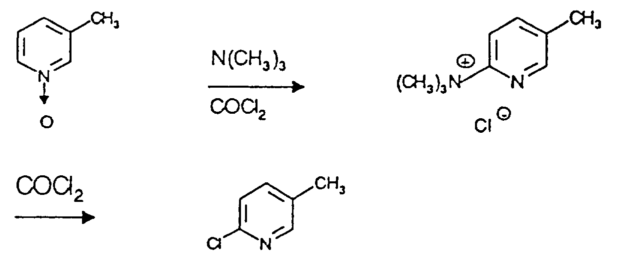

Die Ausgangsverbindung der Formel (II) ist bereits bekannt (vgl. J. Am. Chem. Soc. 76(1954), 1286-1291).

Das in der ersten Stufe des erfindungsgemäßen Verfahrens gebildete Addukt ist durch die Formel (III) wiedergegeben.

Insbesondere steht in Formel (III)

Z⁻ für ein Chlorid- oder ein Acetat-ion.

Die Addukte der Formel (III) sind noch nicht aus der Literatur bekannt und sind als neue chemische Verbindungen Gegenstand der vorliegenden Patentanmeldung.

In der ersten Stufe des erfindungsgemäßen Verfahrens wird weiterhin eine elektrophile Verbindung eingesetzt. Thionylchlorid, Sulfurylchlorid, Phosgen, Thionylchlorid, Sulfurylchlorid, Benzolsulfonsäurechlorid und p-Toluolsulfonsäurechlorid werden als elektrophile Einsatzstoffe für das erfindungsgemäße Verfahren besonders bevorzugt.

Das erfindungsgemäße Verfahren wird in der zweiten Stufe unter Verwendung eines Chlorierungsmittels durchgeführt.

Hydrogenchlorid wird als Chlorierungsmittel in der zweiten Stufe des erfindungsgemäßen Verfahrens besonders bevorzugt.

Das erfindungsgemäße Verfahren wird in der zweiten Stufe gegebenenfalls in Gegenwart eines Alkylchlorids durchgeführt. Bevorzugte Alkylchloride sind Methylchlorid, Ethylchlorid, Propylchlorid und Butylchlorid, insbesondere Methylchlorid.

Das erfindungsgemäße Verfahren wird in der ersten Stufe vorzugsweise unter Verwendung eines Verdünnungsmittels durchgeführt. Als Verdünnungsmittel kommen praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutylketon, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren wird gegebenenfalls auch in der zweiten Stufe in Gegenwart eines Verdünnungsmittels durchgeführt. Als Verdünnungsmittel kommen hierbei neben Wasser praktisch alle inerten organischen Lösungsmittel in Betracht. Hierzu gehören vorzugsweise Alkohole wie Methanol, Ethanol, n- und i-Propanol, Carbonsäuren wie Ameisensäure, Essigsäure und Propionsäure, sowie die oben für die erste Stufe des erfindungsgemäßen Verfahrens angegebenen Solventien.

Die erste Stufe des erfindungsgemäßen Verfahrens wird falls Trimethylamin als organische Stickstoffbase eingesetzt wird, im allgemeinen im Temperaturbereich zwischen -50°C und +120°C, vorzugsweise zwischen -30°C und +80°C durchgeführt. Falls andere organische Stickstoffbasen als Trimethylamin eingesetzt werden, wird im allgemeinen im Temperaturbereich von -30°C bis +100°C, vorzugsweise zwischen -15°C und +10°C gearbeitet.

Die erste Stufe des erfindungsgemäßen Verfahrens wird im allgemeinen bei Normaldruck durchgeführt. Es ist jedoch auch möglich, unter erhöhtem oder vermindertem Druck, im allgemeinen zwischen 0,1 und 100 bar, zu arbeiten.

Zur Durchführung der ersten Stufe des erfindungsgemäßen Verfahrens setzt man auf 1 Mol 3-Methyl-pyridin-1-oxid der Formel (II) im allgemeinen zwischen 1 und 10 Mol, vorzugsweise zwischen 2 und 5 Mol, organische Stickstoffbase und zwischen 1 und 10 Mol, vorzugsweise zwischen 1 und 5 Mol, elektrophile Verbindung ein.

In einer bevorzugten Ausführungsform der ersten Stufe des erfindungsgemäßen Verfahrens wird das 3-Methyl-pyridin-1-oxid der Formel (II) in einem Verdünnungsmittel vorgelegt und nach Abkühlen werden nacheinander zuerst die organische Stickstoffbase und dann die elektrophile Verbindung langsam unter Rühren eindosiert.

Nach dem Ende der Umsetzung werden gegebenenfalls die leichter flüchtigen Komponenten unter vermindertem Druck abdestilliert. Das Roh-Zwischenprodukt, welches im wesentlichen das Addukt der Formel (III) enthält, kann auf übliche Weise, z.B. durch Säulenchromatographie gereinigt oder auch roh in die zweite Stufe eingesetzt werden.

Vorzugsweise wird das Roh-Zwischenprodukt als solches - gegebenenfalls auch ohne Entfernen des Verdünnungsmittels - in die zweite Stufe eingesetzt.

Die zweite Stufe des erfindungsgemäßen Verfahrens wird im allgemeinen im Temperaturbereich zwischen 10° und 150°C, vorzugsweise zwischen 20°C und 120°C, insbesondere zwischen 30°C und 110°C, durchgeführt.

Die zweite Stufe des erfindungsgemäßen Verfahrens wird im allgemeinen bei Normaldruck durchgeführt. Es ist jedoch auch möglich, unter erhöhtem oder vermindertem Druck, im allgemeinen zwischen 0,01 und 200 bar, zu arbeiten.

Die Aufarbeitung kann auf übliche Weise durchgeführt werden. Beispielsweise wird die Rohsubstanz mit einem mit Wasser praktisch nicht mischbaren organischen Lösungsmittel digeriert, mit Wasser gewaschen und getrocknet. Nach sorgfältigem Abdestillieren des organischen Lösungsmittel unter vermindertem Druck verbleibt ein Rückstand, welcher im wesentlichen das Produkt der Formel (I) enthält.

Das nach dem erfindungsgemäßen Verfahren herstellbare 2-Chlor-5-methyl-pyridin ist als Zwischenprodukt für Pharmazeutika bekannt (vgl. DE-A 2812585), kann aber auch als Zwischenprodukt für Insektizide eingesetzt werden (vgl. DE-A 2630046).

### Herstellungsbeispiele:

### Beispiel 1

1. Stufe:

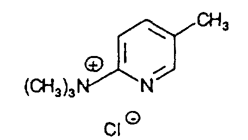

90 g (0,825 Mol) 3-Methyl-pyridin-1-oxid werden in 540 ml Methylenchlorid vorgelegt und das Gemisch wird auf 0°C abgekühlt. Bei dieser Temperatur werden 195 g (3,3 Mol) Trimethylamin einkondensiert. Dann werden 245 g (2,63 Mol) Phosgen eingeleitet, wobei die Temperatur durch intensives Kühlen bei 0°C gehalten wird. Nach Beendigung der Umsetzung wird überschüssiges Phosgen bei 20°C im Wasserstrahlvakuum entfernt. Der verbleibende Rückstand wird durch Säulenchromatographie (Kieselgel; Eluens: Methylenchlorid/Methanol, Vol. 10:1,5) gereinigt.

Man erhält 188 g eines Produktgemisches, welches gemäß ¹H-NMR-Spektrum 102 g (66% der Theorie) Trimethyl-(5-methyl-pyridin-2-yl)-ammoniumchlorid enthält.

NMR-Daten: ¹H-NMR (300 MHz, D₆-DMSO) δ/ppm = 2,41 (3H, Singulett); 3,64 (9H, Singulett); 8,06 (2H, AB-System, J_{AB} = 8,4 Hz); 8,5 (1H, Singulett)

### Vergleichsbeispiel

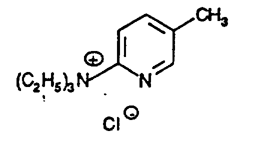

90 g (0,825 Mol) 3-Methyl-pyridin-1-oxid werden in 540 ml Methylenchlorid vorgelegt und das Gemisch wird auf 0°C abgekühlt. Bei dieser Temperatur werden 334 g (3,3 Mol) Triethylamin zugegeben. Dann werden 245 g (2,63 Mol) Phosgen eingeleitet, wobei die Temperatur durch intensives Kühlen bei 0°C gehalten wird. Nach Beendigung der Umsetzung wird überschüssiges Phosgen bei 20°C im Wasserstrahlvakuum entfernt. Der verbleibende Rückstand wird durch Säulenchromatographie (Kieselgel, Eluens: Methylenchlorid/Methanol, Vol. 10: 1,5) gereinigt.

Man erhält 173 g eines Produktgemisches, welches gemäß ¹H-NMR-Spektrum 104 g (55% der Theorie) Triethyl-(5-methyl-pyridin-2-yl)-ammoniumchlorid enthält.

¹H-NMR (300 MHz, CDCl₃)/ppm = 1,20 (9H, Triplett, J = 7,2 Hz), 2,44 (3H, Singulett), 4,14 (6H, Quartett, J = 7,2 Hz), 7,98 (1H, Multiplett, J_{AB} = 8,4 Hz), 8,34 (1H, Quartett, J = 0,9 Hz), 8,63 (1H, Dublett, J_{AB} = 8,7 Hz)

### Beispiel 2

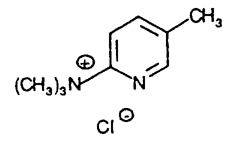

19,5 g (0,331 Mol) Trimethylamin werden bei -10°C kondensiert und bei -5°C zu einer Lösung von 8,8 g (0,0807 Mol) 3-Methyl-pyridin-1-oxid in 100 ml Methylenchlorid gegeben. Dann werden bei dieser Temperatur 6,9 ml (0,097 Mol) Thionylchlorid in 10 ml Methylenchlorid innerhalb 30 Minuten eingetropft. Die Temperatur wird während dieses Vorgangs unter 0°C gehalten. Die resultierende gelbe Lösung läßt man auftauen und über Nacht bei Raumtemperatur nachrühren.

Man erhält eine goldgelbe Lösung, die bei 20-25°C am Rotationsverdampfer eingeengt wird. Es fallen 24,5 g eines schmierigen Feststoffes an, der laut ¹H-NMR ca. 14,5 g (96% der Theorie) Trimethyl-(5-methyl-pyridin-2-yl)-ammoniumchlorid enthält.

¹H-NMR: (300 MHz, D₆-DMSO)/ppm = 2,41 (3H, Singulett); 3,64 (9H, Singulett), 8,06 (2H, AB-System, J_{AB} = 8,4 Hz), 8,5 (1H, Singulett)

### Beispiel 3

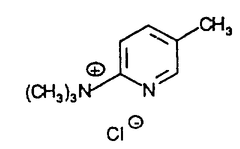

20,5 g (0,348 Mol) Trimethylamin werden bei -10°C kondensiert und anschließend bei -5°C zu einer Lösung von 8,8 g (0,0807 Mol) 3-Methyl-pyridin-1-oxid in 110 ml Methylenchlorid gegeben. Dann wird bei dieser Temperatur eine Lösung von 21,3 ml (0,2421 Mol) Sulfurylchlorid in 20 ml Methylenchlorid eingetropft. Es bildet sich ein weißer, feiner Niederschlag. Man läßt innerhalb einer Stunde auf Raumtemperatur auftauen und anschließend noch 2 Stunden bei dieser Temperatur nachrühren. Der Niederschlag löst sich in dieser Zeit vollständig auf. Die resultierende goldgelbe Lösung wird über Nacht stehengelassen und anschließend am Rotationsverdampfer eingeengt.

Man erhält 48,7 g eines schmierigen Feststoffes, der laut ¹H-NMR 11,6 g (77% der Theorie) Trimethyl-(5-methyl-pyridin-2-yl)-ammoniumchlorid enthält.

### Beispiel 4

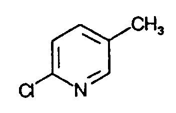

109 g (1,0 Mol) 3-Methyl-pyridin-1-oxid werden in 800 ml 1,2-Dichlorethan vorgelegt und das Gemisch wird auf -30°C abgekühlt. Bei dieser Temperatur werden 130 g (2,2 Mol) Trimethylamin einkondensiert und dann werden unter ständigem Kühlen 212 g (1,2 Mol) Benzolsulfonsäurechlorid innerhalb von 2 Stunden eindosiert. Nach Entfernen der Kühlung wird das Reaktionsgemisch 15 Stunden bei 20°C gerührt. Dann wird das Gemisch auf 35°C erwärmt und bei dieser Temperatur Hydrogenchlorid eingeleitet, bis nach dünnschichtchromatographischer Analyse die Umsetzung abgeschlossen ist (Dauer: ca. 32 Stunden). Zur Aufarbeitung wird mit 20%iger Natronlauge ein alkalischer pH-Wert eingestellt und mit Dichlormethan extrahiert. Die organische Phase wird mit Natriumsulfat getrocknet und filtriert. Vom Filtrat wird im Wasserstrahlvakuum das Lösungsmittel sorgfältig abdestilliert.

Man erhält 136 g eines Rohprodukts, das nach gaschromatographischer Analyse 78% 2-Chlor-5-methyl-pyridin enthält.

Ausbeute: 84% der Theorie.

## Patentansprüche

1. Verfahren zur Herstellung von 2-Chlor-5-methyl-pyridin der Formel 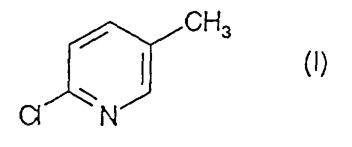 dadurch gekennzeichnet, daß man in einer ersten Stufe 3-Methyl-pyridin-1-oxid der Formel (II) 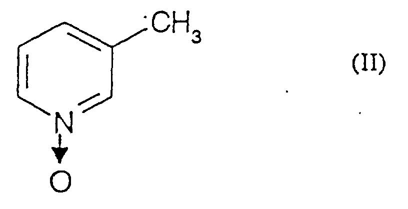 mit Trimethylamin und einer elektrophilen Verbindung aus der Reihe Phosgen, Thionylchlorid, Sulfurylchlorid, Benzolsulfonsäurechlorid und p-Toluolsulfonsäurechlorid gegebenenfalls in Gegenwart eines Verdünnungsmittels,
zur Verbindung der allgemeinen Formel (III) 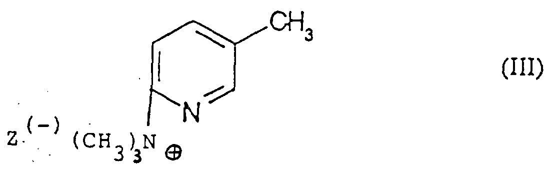 in welcher
Z^{⊖} für ein aus der elektrophilen Verbindung gebildetes Anion steht, umsetzt,
und diese Verbindung (III) gegebenenfalls
als Rohzwischenprodukt isoliert oder gegebenenfalls weiter reinigt,
und in einer zweiten Stufe mit Chlorwasserstoff als Chlorierungsmittel gegebenenfalls in Gegenwart eines Alkylchlorids und gegebenenfalls in Gegenwart eines Verdünnungsmittels bei Temperaturen zwischen 10°C und 150°C umsetzt.

2. Verfahren gemäß Anspruch 1, dadurch gekennzeichnet, daß die erste Reaktionsstufe im Temperaturbereich zwischen -50°C und +120°C durchgeführt wird.

3. Verfahren gemäß Anspruch 1, dadurch gekennzeichnet, daß die zweite Reaktionsstufe (Chlorierung) bei Temperaturen zwischen 20°C und 120°C durchgeführt wird.

4. Verfahren gemäß Anspruch 1, dadurch gekennzeichnet, daß man auf 1 Mol 3-Methyl-pyridin-1-oxid (II) 1 bis 10 Mol Trimethylamin und 1 bis 10 Mol elektrophile Verbindung einsetzt.

5. Verfahren gemäß Anspruch 1, dadurch gekennzeichnet, daß man auf 1 Mol 3-Methyl-pyridin-1-oxid (II) 2 bis 5 Mol Trimethylamin und 2 bis 5 Mol elektrophile Verbindung einsetzt.

6. Verfahren gemäß Anspruch 1, dadurch gekennzeichnet, daß man in der ersten Stufe des erfindungsgemäßen Verfahrens das 3-Methyl-pyridin-1-oxid (II) in einem inerten organischen Verdünnungsmittel vorlegt und nach dem Abkühlen nacheinander zuerst Trimethylamin und dann die elektrophile Verbindung langsam unter Rühren eindosiert.

7. Verbindung der Formel

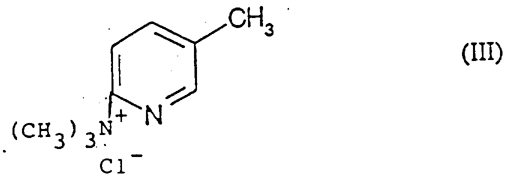

## Claims

1. Process for the preparation of 2-chloro-5-methylpyridine of the formula 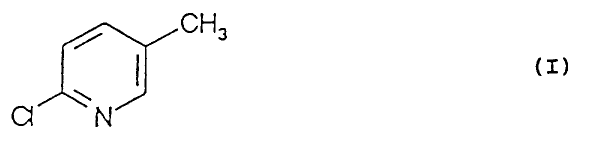 characterised in that in a first stage 3-methyl-pyridine 1-oxide of the formula (II) 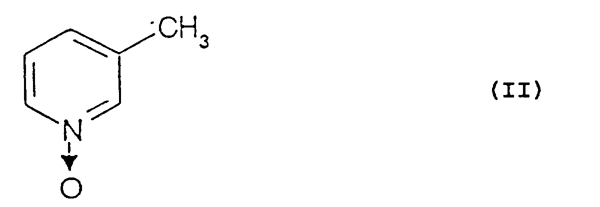 is reacted with trimethylamine and an electrophilic compound from the series consisting of phosgene, thionyl chloride, sulphuryl chloride, benzenesulphonyl chloride and p-toluenesulphonyl chloride, optionally in the presence of a diluent, to give the compound of the formula (III) 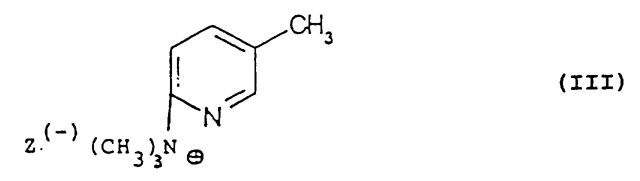 in which
Z^{⊖} represents an anion formed from the electrophilic compound,
and this compound (III) is optionally
isolated as a crude intermediate product or optionally further purified,
and in a second stage is reacted with hydrogen chloride as chlorinating agent, optionally in the presence of an alkyl chloride and optionally in the presence of a diluent, at temperatures between 10°C and 150°C.

2. Process according to Claim 1, characterised in that the first reaction stage is performed in the temperature range between -50°C and +120°C.

3. Process according to Claim 1, characterised in that the second reaction stage (chlorination) is performed at temperatures between 20°C and 120°C.

4. Process according to Claim 1, characterised in that 1 to 10 mol of trimethylamine and 1 to 10 mol are used per mole of 3-methyl-pyridine 1-oxide (II).

5. Process according to Claim 1, characterised in that 2 to 5 mol of trimethylamine and 2 to 5 mol of electrophilic compound are used per mole of 3-methyl-pyridine 1-oxide (II).

6. Process according to Claim 1, characterised in that in the first stage of the process according to the invention the 3-methyl-pyridine 1-oxide (II) is initially introduced into an inert organic diluent and, after cooling, first trimethylamine and then the electrophilic compound are slowly metered in one after the other with stirring.

7. Compound of the formula

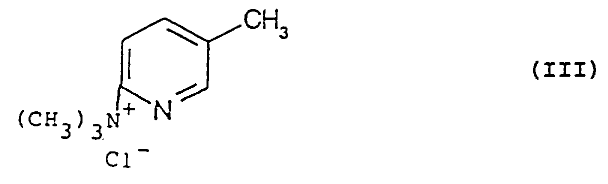

## Revendications

1. Procédé de production de 2-chloro-5-méthylpyridine de formule 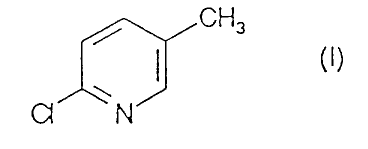 caractérisé en ce qu'on fait réagir dans une première étape le 1-oxyde de 3-méthylpyridine de formule (II) 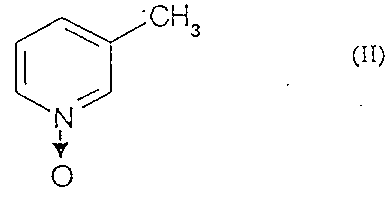 avec la triméthylamine et un composé électrophile de la série phosgène, chlorure de thionyle, chlorure de sulfuryle, chlorure d'acide benzènesulfonique et chlorure d'acide p-toluènesulfonique, éventuellement en présence d'un diluant,
pour obtenir un composé de formule générale (III) 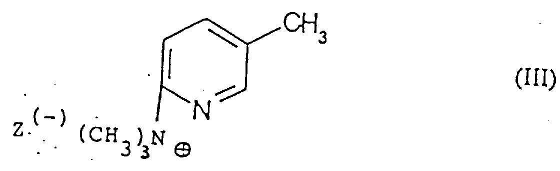 dans laquelle
Z^{⊖} représente un anion formé à partir du composé électrophile,
et, le cas échéant, on isole comme produit intermédiaire brut ce composé (III) et on poursuit éventuellement sa purification,
et dans une seconde étape, on le fait réagir à des températures comprises entre 10°C et 150°C avec du chlorure d'hydrogène comme agent de chloration, éventuellement en présence d'un chlorure d'alkyle et en la présence éventuelle d'un diluant.

2. Procédé suivant la revendication 1, caractérisé en ce qu'on conduit la première étape de la réaction dans la plage de températures de -50°C à +120°C.

3. Procédé suivant la revendication 1, caractérisé en ce qu'on conduit la seconde étape (chloration) de la réaction à des températures comprises entre 20°C et 120°C.

4. Procédé suivant la revendication 1, caractérisé en ce qu'on utilise 1 à 10 moles de triméthylamine et 1 à 10 moles de composé électrophile par mole de 1-oxyde de 3-méthylpyridine (II).

5. Procédé suivant la revendication 1, caractérisé en ce qu'on utilise 2 à 5 moles de triméthylamine et 2 à 5 moles de composé électrophile par mole de 1-oxyde de 3-méthylpyridine (II).

6. Procédé suivant la revendication 1, caractérisé en ce que, dans la première étape du procédé conforme à l'invention, on charge préalablement le 1-oxyde de 3-méthylpyridine (II) dans un diluant organique inerte et, après refroidissement, on y incorpore lentement et successivement, en agitant, tout d'abord la triméthylamine, puis le composé électrophile.

7. Composé de formule
